# EUROPEAN PATENT APPLICATION

(11) **EP 3 766 963 A1**
(43) Date of publication of application: **20.01.2021**
(21) Application number: 19186342.2
(22) Date of filing: 15.07.2019
(51) Int. Cl.: C12N 5/0775, A61K 35/28

(54) **METHOD OF CULTURING MESENCHYMAL STEM CELLS**

(71) Applicant: Universität Leipzig, 04109 Leipzig (DE)
(72) Inventor: SAVKOVIC, Vuk, 04315 Leipzig (DE); LI, Hanluo, 04103 Leipzig (DE); SIMON, Jan-Christoph, 04416 Markkleeberg (DE); ETZ, Christian, 04103 Leipzig (DE); SCHNEIDER, Marie, 04155 Leipzig (DE)
(74) Representative: Lederer & Keller Patentanwälte Partnerschaft mbB

(57) **Abstract**

The present invention provides methods for expanding mesenchymal stem cells obtained from the outer root sheath of hair follicles. The invention further provides methods for differentiating the expanded cells into differentiated cells and tissues, and cells obtainable by the methods of the invention.

## Description

The present invention provides methods for expanding mesenchymal stem cells obtained from the outer root sheath of hair follicles (MSCORS). The invention further provides methods for differentiating the expanded cells into differentiated cells and tissues, and cells obtainable by the methods of the invention.

### BACKGROUND

Mesenchymal stem cells (MSCs) are a group of stem cells residing in the stromal tissue with multipotent differentiation capacities, structural support, immunosuppressive effects and paracrine functions. As the most important source of somatic tissue stem cells, MSCs maintain and regenerate entire body systems via differentiation and paracrine functions, and have been found in various tissues, including bone marrow, adipose tissue, peripheral blood, lung, and hair follicles, as well as in neonatal tissues such as placenta and umbilical cord. MSCs are a prevalent source of regenerative stem cells for clinical applications, well known for their multipotency, autologous applicative potential and accessibility.

MSCs, especially humans MSCs, are therefore a promising candidate for cosmetic and clinical regenerative treatments for purposes of aesthetic, structural or functional improvement as well as for the treatment of a disorder affecting cells, tissues, organs or organ systems, in particular for bone repair, cartilage repair and heart repair, as well as for further disorders of tissues and organs.

Bone marrow MSC (BMMSC) are most popular and widely used stem cell source from human body. BMMSC are usually isolated from iliac crest via femoral puncture. This isolation method requires a surgical operation with anesthesia, with potential post-biopsy complications. Adipose MSC (ADMSC) are usually isolated from subcutaneous fat via lipoaspiration and lipectomy, which also requires a surgical operation with anesthesia, which is simpler and less invasive than isolating BMMSC. ADMSC are more abundantly available than BMMSC. Each of these sources of adult mesenchymal stem cells has an innate problem of invasive biopsy needed to extract the cells.

Umbilical Cord MSC (UCB-MSC) are isolated from umbilical cord blood or from cavernous Wharton's jelly tissue, which is rich in stem cells. The isolated stem cells harbor the most primitive properties of MSCs. Those MSCs are less abundant compared to bone marrow and adipose tissue, and they lack the capacity for adipogenesis. However, after isolation, the UCB-MSC can be amplified and cryopreserved for possible autologous or allogeneic use in the treatment of hematopoietic diseases.

A novel source of stem cells, the outer root sheath of hair follicle (ORS), offers non-invasively obtainable MSCs from autologous somatic source. The ORS as a stem cell reservoir is a heterogeneous pool that contains stem cells with various developmental potential, including MSCs. Compared to those aforementioned sampling procedures, hair follicle as an MSC source has the advantage of being extracted from the body without any pain or discomfort or risk of infection, by simple hair plucking using tweezers. The sampling procedure leaves no sequential wounds or scars and the extracted hairs re-grow.

WO 2013/060899 A2 and Savkovic et al. (2012) Experimental Dermatology 21: 948-976 describe methods for generating melanocytes from the hair follicle outer root sheath using plucked hairs from donors. Upon isolation, the hair follicle and cells derived therefrom are cultured in medium which promotes differentiation into melanocytes. WO 2013/060899 A2 and Savkovic et al. do not aim at expanding MSCs without differentiation.

A low-efficiency procedure for extracting MSCs from a suspension of ORS cells has been previously published (Ma D, Kua JE, Lim WK, Lee ST, Chua AW (2015) Cytotherapy 17(8):1036-51).

Li et al. (2015) Cell Tissue Res 362: 69-82 investigates the feasibility of human hair follicle-derived mesenchymal stem cells/CultiSpher®-G constructs in regenerative medicine. The inventors have carried out the method described in Li et al. and found that it yields an insufficient number of MSCs per hair follicle (see Example 3 *infra*)*.* In addition, the cells of Li et al. appear to contain a substantial amount of fibroblasts, as the dermal papilla was kept in Li et al.

Zhang et al. (2013) International Journal of Molecular Medicine 31: 913-921 asserts that high proliferation and multipotent potential of human hair follicle-derived MSCs can be maintained by growth factors. The inventors have carried out the method described in Zhang et al. and found that it yields an insufficient number of MSCs per hair follicle (see Example 3 *infra*)*.* In addition, the cells of Zhang et al. appear to contain a substantial amount of fibroblasts, as the dermal papilla was kept in Zhang et al.

Wang et al. (2013) Stem Cell Rev and Rep 9: 451-460 investigates induced pluripotent stem cells from human hair follicle MSCs. The inventors have carried out the method described in Wang et al. and found that it yields an insufficient number of MSCs per hair follicle (see Example 3 *infra*)*.* In addition, the cells of Wang et al. appear to contain a substantial amount of fibroblasts, as the dermal papilla was kept in Wang et al.

Obtaining a sufficient number of MSCs is important. For a clinical cell therapy the required number of cells for a single dose is typically at least 10 million cells. Also for research purposes several million cells are required to carry out a set of experiments (e.g. cell viability, cell growth, flow cytometry, RT-PCR, different lineages of differentiation). On the other hand, obtaining a sufficient number of MSCs from non-invasively plucked hair follicle ORS is difficult.

There is a need for sources of MSCs which are easily obtainable and can be efficiently proliferated to yield a high number of MSCs which can be used for differentiation into target cells and tissues. Such differentiated cells and tissues can be used in regenerative medicine.

### SUMMARY OF THE INVENTION

The inventors surprisingly found that MSCs with a very high, unexpected proliferative potential can be obtained from hair follicles if the bulb of the hair follicles is removed, the follicles are treated with a collagenase and then cultured on a membrane. In particular, the method developed by the inventors allows the successful proliferation of MSCs from a high percentage of hair follicles used. This enables the expansion to more than 6 million MSCs from, e.g., only 45 hair follicles within four to six weeks. The present invention therefore relates to the subject matter defined in the following items [1] to [48]:
[1] A method for generating mesenchymal stem cells comprising the steps of:
   (i) removing the bulb of an epilated hair, preferably of an epilated human hair, more preferably of an epilated human anagen hair;
   (ii) incubating the remaining part of the epilated hair with a collagen degrading agent to obtain a hair follicle comprising stem cells and an at least partially degraded extracellular matrix;
   (iii) culturing the hair follicle obtained from step (ii) on a liquid-permeable membrane in a first medium to allow outgrowth of the stem cells from the hair follicle, under conditions that induce proliferation of the stem cells without inducing their differentiation; and
   (iv) further culturing the stem cells obtained from step (iii) on a solid support in a second medium that induces proliferation of the stem cells without inducing their differentiation.
[2] The method of item [1], wherein a plurality of epilated hairs are used to generate the mesenchymal stem cells.
[3] The method of item [2], wherein at least 10 epilated hairs are used to generate the mesenchymal stem cells.
[4] The method of item [2], wherein at least 20 epilated hairs are used to generate the mesenchymal stem cells.
[5] The method of item [2], wherein at least 30 epilated hairs are used to generate the mesenchymal stem cells.
[6] The method of item [2], wherein at least 40 epilated hairs are used to generate the mesenchymal stem cells.
[7] The method of item [2], wherein 10 to 100 epilated hairs are used to generate the mesenchymal stem cells.
[8] The method of any one of items [2] to [7], wherein at least 50% of the epilated hairs result in a proliferating culture of mesenchymal stem cells.
[9] The method of any one of items [2] to [7], wherein at least 70% of the epilated hairs result in a proliferating culture of mesenchymal stem cells.
[10] The method of any one of items [2] to [7], wherein at least 90% of the epilated hairs result in a proliferating culture of mesenchymal stem cells.
[11] The method of any one of the preceding items, comprising, prior to step (i), immersing the epilated hair in medium comprising L-glutamine and optionally at least one antibiotic, for 1 to 10 hours, preferably for 2 to 5 hours.
[12] The method of any one of the preceding items, comprising removing the bulb of the epilated hair.
[13] The method of any one of the preceding items, further comprising, after step (i) and prior to step (ii), washing the remaining part of the epilated hair with a solution comprising a buffer, L-glutamine and optionally at least one antibiotic.
[14] The method of any one of the preceding items, wherein said collagen degrading agent is a collagenase.
[15] The method of item [14], wherein said collagenase is capable of degrading collagen X or collagen V or collagen IV.
[16] The method of any one of the preceding items, comprising, after step (ii) and prior to step (iii), transferring the remaining hair follicle onto the liquid-permeable membrane, preferably a Transwell membrane of 0.4um pore size.
[17] The method of any one of the preceding items, wherein a compartment below the membrane comprises a feeder layer of irradiation or mitomycin-inactivated fibroblasts, preferably human inactivated dermal fibroblasts, and the medium in said compartment is in contact with the bottom side of the membrane.
[18] The method of any one of the preceding items, wherein said first medium comprises serum, preferably fetal bovine serum and most preferably human serum.
[19] The method of any one of the preceding items, wherein said first medium comprises bFGF and/or EGF; and optionally insulin, human transferrin, and/or selenite.
[20]. The method of any one of the preceding items, wherein step (iii) is carried out for 14 to 25 days.
[21] The method of any one of the preceding items, wherein step (iii) is carried out for 18 to 23 days.
[22] The method of any one of the preceding items, comprising, after step (iii) and prior to step (iv), transferring the stem cells obtained from step (iii) to a solid support, preferably a solid support which is not liquid-permeable.
[23] The method of any one of the preceding items, wherein said solid support substantially consists of polystyrene, optionally coated with a polymer promoting cell attachment and proliferation (e.g. supports with the Advanced TC™ polymer modification; Greiner Bio-One).
[24] The method of any one of the preceding items, wherein said second medium comprises serum, preferably fetal bovine serum and most preferably human serum.
25] The method of any one of the preceding items, wherein said second medium comprises bFGF and/or EGF; and optionally insulin, human transferrin, and/or selenite.
[26] The method of any one of the preceding items, wherein the second medium comprises IL-6.
[27] The method of any one of the preceding items, wherein step (iv) comprises culturing the separated stem cells until the number of proliferated stem cells is at least 6x10⁶.
[28] The method of any one of the preceding items, wherein
   - step (iv) is carried out for 21 days to 35 days;
   - step (iv) comprises culturing the stem cells for 8 to 20 days, preferably for 10 to 16 days without passaging the cells ;
   - step (iv) comprises passaging the cells twice, optionally wherein the culturing time after the first passage is from 16 to 26 days, preferable from 18 to 24 days; and/or
   - the stem cells obtained from step (iv) were passaged twice (P2).
[29] The method of any one of the preceding items, wherein steps (iii) and/or (iv) comprise culturing the cells under hypoxic conditions.
[30] The method of any one of the preceding items, wherein the number of mesenchymal stem cells obtained from step (iv) is at least 10⁵ per epilated hair.
[31] The method of any one of the preceding items, wherein the MSCs obtained from step (iv) are positive for the markers CD73, CD90, CD105, CD44, CD133, CD13, CD71, Nestin, N-Cadherin, Fibronectin, Vimentin, Integrin α5 (CD49e) and Integrin αV (CD51), and are negative for the markers CD34, CD45, CD11b, CD19 and HLA-DR.
[32] A method for generating differentiated cells, comprising (a) generating MSCs by a method according to any one of the preceding items, and (b) differentiating the MSCs or the proliferated stem cells obtained from step (iv) of claim 1 to obtain differentiated cells.
[33] The method of item [32], wherein said differentiated cells are selected from the group consisting of cardiomyocytes, chondrocytes, osteoblasts, adipocytes, endothelial cells and smooth muscle cells.
[34] The method of item [31], wherein said differentiated cells are chondrocytes.
[35] The method of item [31], wherein said differentiated cells are cardiomyocytes.
[36] The method of item [31], wherein said differentiated cells are osteoblasts.
[37] The method of item [31], wherein said differentiated cells are adipocytes.
[38] The method of item [31], wherein said differentiated cells are endothelial cells.
[39] The method of item [31], wherein said differentiated cells are smooth muscle cells.
[40] A population of mesenchymal stem cells obtainable by the method of any one of items [1] to [31].
[41] The use of mesenchymal stem cells obtainable by the method of any one of items [1] to [31] for generating differentiated cells, preferably differentiated cells as defined in any one of items [33] to [39].
[42] A mesenchymal stem cell obtainable by the method of any one of items [1] to [31] for use in the treatment of a disorder affecting cells, tissues, organs or organic systems that are structurally or functionally related to mesenchymal cells or structures.
[43] The mesenchymal stem cell for use according to item [42], wherein said disorder is a bone disorder.
[44] The mesenchymal stem cell for use according to item [43], wherein said bone disorder is a bone injury.
[45] The mesenchymal stem cell for use according to item [42], wherein said disorder is a disorder affecting the cartilage.
[46] The mesenchymal stem cell for use according to item [45], wherein said disorder affecting the cartilage is osteoarthritis.
[47] The mesenchymal stem cell for use according to item [42], wherein said disorder is a cardiovascular disorder.
[48] The mesenchymal stem cell for use according to item [42], wherein said disorder is a skin wound of any thickness.

### DESCRIPTION OF THE FIGURES

**Figure 1** depicts plots of a FACS analysis of positive and negative MSC markers in MSCORS (A) compared to ADMSC (B) (Surface biomarker expression of MSCORS and ADMSC using FACS analysis according to the MSC classification Criteria defined by ISCT. FACS plot graphs and histograms of a representative labeling are shown).
**Figure 2** shows the gene expression profile of MSCORS depending on the passage number (Gene expression profile of MSCORS from passage 2 to passage 5 according to the MSC classification Criteria defined by ISCT).
**Figure 3** shows the results of αSMA filament subcellular morphology (Demonstration of semi-quantitative analysis of αSMA-immunostained actin stress fibers using ImageJ, in terms of filament number and length).
**Figure 4** shows the osteogenesis, semi-quantitative analysis of MSCORS and ADMSC (Quantitative analysis of Alizarin Red staining and ALP activity assay using ImageJ for purposes of the osteogenic differentiation assessment).
**Figure 5** depicts the deposition of Calcium concentration of MSCORS and ADMSC (Extracellular calcium phosphate deposition of MSCORS and ADMSC after osteogenic differentiation).
**Figure 6** depicts results from the Angiogenesis Assay Semi-Quantitative Analysis in MSCORS and ADMSC (Tube-Forming Assay of MSCORS and ADMSC demonstrating endothelial differentiation, and quantitative analysis of the anastomosis by the means of the ImageJ software).
**Figure 7** depicts cell numbers across 15 passages of MSCORS cultivation using the procedure described herein (Comparison of MSCORS and D12: empirical Cell Count in each passage).
**Figure 8** depicts results from the comparison of the MSCORS cultivation procedure described herein and Wang for cultivating MSCs from the hair follicle (Comparison of MSCORS and D12: Projected Cell Yield in each passage).

### DETAILED DESCRIPTION

The term mesenchymal stem cell (MSC) as used herein refers to cells having the following properties: First, MSC must be plastic-adherent when maintained in standard culture conditions using tissue culture flasks. Second, ≥95% of the MSC population must express CD105, CD73 and CD90, as measured by flow cytometry. Additionally, these cells must lack expression (≤2% positive) of CD45, CD34, CD14 or CD11β, CD79α or CD19 and HLA class II. Third, the cells must be able to differentiate at least to osteoblasts, adipocytes and chondroblasts under standard in vitro differentiating conditions.

The present invention relates to a method for generating MSCs comprising the steps of:
(i) removing the bulb of an epilated hair;
(ii) incubating the remaining part of the epilated hair with a collagen degrading agent to obtain a hair follicle comprising stem cells and an at least partially degraded extracellular matrix;
(iii) culturing the hair follicle obtained from step (ii) on a liquid-permeable membrane in a first medium to allow outgrowth of the stem cells from the hair follicle, under conditions that induce proliferation of the stem cells without inducing their differentiation; and
(iv) further culturing the stem cells obtained from step (iii) on a solid support in a second medium that induces proliferation of the stem cells without inducing their differentiation.

The hair referred to in the method of the present invention is preferably a human hair, more preferably a human anagen hair. The preferred embodiments concerning the hair source are applicable to any aspect of the invention.

The method of the invention optionally comprises the step of plucking one or more hairs from the scalp of an individual to obtain the epilated hair(s) used in step (i). The hairs may be plucked from any region of the scalp, preferably from the temporal or occipital region of the scalp. Up to five hairs may be held close to the root with disinfected forceps and pulled in the direction of the hair growth. Plucked hair follicles may be immersed in a suitable medium (e.g. DMEM) optionally with antibiotics and/or L-Glutamine (e.g. for 2-4 hours) at room temperature (e.g. 25°C). Alternatively, the hairs can be stored in phosphate-buffered saline (PBS) until preparation without addition of calcium or magnesium. Hereinafter the abbreviation of PBS refers to phosphate-buffered saline without addition of calcium or magnesium.

The skilled person is able to adapt the amount of epilated hairs depending on the number of MSCs required. In one embodiment, the follicles of 1 to 500, preferably the follicles of 10 to 80, more preferably the follicles of 30 to 60 epilated hairs, most preferably 15 to 20 hairs from one donor, are removed in step (i) of the method of the present invention.

The method according to the present invention comprises removing the bulb of an epilated hair. The "bulb" of an epilated hair in context of the present invention is the proximal part of the hair root, which predominantly contains Dermal Papilla cells (DP cells) which are phenotypically similar to fibroblasts, yet limited in differentiation and proliferation, as well as differentiated cells (such as melanocytes and keratinocytes). In a preferred embodiment also the distal part of the hair shaft, also called 'the bulge' is cut off if not lost during epilation. In a preferred embodiment only the mid-part of the epilated hair root is used. The mid-part of an epilated hair in context with the present invention is preferably the morphological unit between the bulb (proximal part) and the sebaceous gland channel. In context with the present invention the terms epilated hair and epilated hair follicle are used interchangeably. Preferably, the proximal part of hair follicle and the excess hair shaft are cut off before the vigorous rinsing.

Typically, after step (i) and prior to step (ii), the remaining part of the epilated hair is washed using washing medium (e.g. PBS + antibiotics + L-glutamine), preferably at least 5 times, more preferably at least 10 times. Each washing cycle is typically conducted for 5 to 20 minutes, preferably for 10-15 min. This step eliminates germ/bacteria contamination in the culture. Preferably, a large volume of washing medium is used, e.g. 10ml in 50ml conical tubes. After each wash, the hairs are shortly left in the wash medium and all of the supernatant is carefully aspirated and discarded, without disturbing or losing the hair follicles. The washing step has the advantage that antibiotics can be omitted or reduced in the medium to be used in the subsequent steps. In a specific embodiment, only media without antibiotics are used in the subsequent method steps (ii), (iii) and (iv).

The remaining epilated hairs are then contacted with a collagen degrading agent. In accordance with the present invention the collagen degrading agent is used for supporting the migration of cells. Without wishing to be bound to a specific theory, it is believed that the collagen degrading agent degrades the collagen fiber complex, and thereby loosens the compact tissue of hair follicle ORS. This facilitates the outgrowth of MSCORS.

Collagen degrading agents are known to the skilled person. Collagen degrading agents in context of the present invention are all agents which digest collagen into smaller subunits. Digestion of collagen weakens the extracellular matrix and releases the cells, including stem cells, precursors and differentiated cells. In a preferred embodiment of the present invention the collagen degrading agent is capable of degrading a collagen selected from the group consisting of collagen I, collagen IV, collagen V, collagen X and combinations thereof. In a preferred embodiment the collagen degrading agent is an enzyme, preferably a collagenase. The collagenases are preferably able to degrade more than one type of collagen. In a particularly preferred embodiment the collagen degrading agent is capable of degrading collagen X. In another preferred embodiment the collagen degrading agent is used at a concentration of about 5 mg/ml. For example, washed hair follicles may be carefully transferred onto a solid support (e.g. with a 100 µm mesh), which supports the hair follicles, and digested with a suitable agent (e.g. with 5mg/ml Collagenase for about 12 minutes).

In a preferred embodiment of the method of the invention, the method comprises a further step between step (ii) and (iii) of washing the hair follicle with a washing solution after incubation with the collagen degrading agent.

Suitable washing solutions are well known in the art and can be chosen by those of skills in the art. In a preferred embodiment the washing solution is Dulbecco's Modified Eagle Medium (DMEM). In a further embodiment the washing solution comprises an antibiotic, preferably selected from the group consisting of Gentamycin, Amphotericin B, Penicillin, Streptomycin, Neomycin, Carbenicillin, Penicillin G, Ampicillin, Polymyxin-B, tetracycline, Ciprofloxacin, Lincomycin, Spectinomycin, Cabenicilin, Thiostrepton, Ceftacidin, Apramycin, Vancomycin, Tobramycin, Rifampycin, and Hygromycin. The washing solution may comprise an antibiotic, preferably selected from the group consisting of Gentamycin, Amphotericin B, Penicillin, and Streptomycin.

The skilled person is able to determine suitable concentrations of antibiotics for the washing solution. Preferred antibiotics and preferred concentrations in parentheses are given in the following: Penicillin, e.g. (50 U/ml to 150 U/ml, preferably 100 U/ml), Streptomycin-Sulfate (50 pg/ml to 20 mg/ml, preferably 100 µg/ml), Gentamycin-Sulfate (5 µg/ml to 3000 µg/ml, preferably 50 ug/ml), Amphotericin B (0.25 µg/ml to 30 µg/ml, preferably 2.5 µg/ml), (25 µg/ml to 600 µg/ml, preferably 50 µg/ml), (50 pg/ml to 10000 µg/ml, preferably 100 µg/ml), (25 µg/ml to 3000 µg/ml, preferably 50 ug/ml), Ampicillintrihydrat (50 U/ml to 200 U/ml, preferably 100 U/ml), Carbenicillin, (50 U/ml to 200 U/ml, preferably 100 U/ml) (50 U/ml to 200 U/ml, preferably 100 U/mL), Polymyxin-B-Sulfate (25 µg/ml to 3000 µg/ml, preferably 100 µg/ml), (2 µg/ml to 80 µg/ml, preferably 10 µg/ml), 7-Hydroxy-Tetracyclin (each 2 to 25 µg/ml, preferably 5 µg/ml), (5 to 35 µg/ml, preferably 10 µg/ml), Erythromycin (50 µg/ml to 300 µg/ml, preferably 100 µg/ml), (2 µg/ml to 300 µg/ml, preferably 10 µg/ml), (2 µg/ml to 30 µg/ml, preferably 5 µg/ml), Ciprofloxacin (1 µg/ml to 10 µg/ml), Lincomycin (about 50 µg/ml), Spectinomycin) (5 µg/ml to 50 µg/ml, preferably 10 µg/ml), Carbenicilin (about 100 U/ml), Thiostrepton (about 25 µg/ml), Ceftacidin-Hydrate (about 100 µg/ml), Apramycin (2.5 µg/ml to 25 µg/ml), Vancomycin-Hydrochloride (100 µg/ml), Tobramycin (4 µg/ml to 100 µg/ml, preferably 80 µg/ml), Rifampycin (about 400 µg/ml), Hygromycin (about 200 µg/ml).

In a preferred embodiment the washing solution comprises Penicillin at a concentration of 50 U/ml to 150 U/ml, preferably 100 U/ml, Streptomycin-Sulfate (50 pg/ml to 20 mg/ml, preferably 100 µg/ml), Gentamycin, preferably at a concentration of 10 µg/ml to 100 µg/ml, more preferably at a concentration of 25 µg/ml to 75 µg/ml, even more preferably at a concentration of 50 µg/ml. In a further preferred embodiment the washing solution comprises Amphotericin B, preferably at a concentration of 1 µg/ml to 20 µg/ml, more preferably at a concentration of 5 µg/ml to 15 µg/ml, even more preferably at a concentration of 10 µg/ml. In a preferred embodiment the washing solution comprises four antibiotics, preferably Penicillin, Streptomycin, Gentamycin and Amphotericin B.

In yet a further preferred embodiment the washing solution comprises DMEM, Gentamycin at a concentration of about 50 µg/ml and Amphotericin B at a concentration of about 10 µg/ml. In a most preferred embodiment the washing solution substantially consists of DMEM, Gentamycin at a concentration of 50 µg/ml and Amphotericin B at a concentration of 10 µg/ml.

In another preferred embodiment the washing solution comprises Penicillin and Streptomycin.

In yet another preferred embodiment the washing solution does not comprise an antibiotic.

The hair follicles obtained from step (ii) are then cultured in a first medium which induces proliferation of stem cells but does not induce their differentiation (hereinafter referred to as "first medium"). This step preferably comprises transferring the hair follicles onto a liquid-permeable support, e.g. a membrane such as a Transwell membrane (available from Corning). Preferably, the liquid-permeable membrane separates a lower compartment from an upper compartment such that cells cannot pass the membrane, whereas liquids can permeate the membrane. For example, the hair follicles may be carefully transferred onto Transwell membranes of 6-well plate format, with about 10 hairs in each of the six wells. Hair follicles are preferably carefully seeded evenly onto the membrane with interval space to allow the cell outgrowth. During the transfer, the forceps are preferably only allowed to clamp the shortened end of the hair shafts, hereby not disturbing the ORS tissue.

The first medium does not contain agents which promote differentiation of MSCs into differentiated cells in an amount sufficient to promote differentiation of MSCs into differentiated cells. In particular, the first medium preferably does not contain separately added agents which promote differentiation of MSCs into cardiomyocytes, chondrocytes, osteoblasts, adipocytes, endothelial cells and smooth muscle cells. Preferably the first medium contains substantially no
- β-adrenergic receptor ligands, such as epinephrine and derivatives thereof;
- dexamethasone,
- ascorbic acid,
- β-giycerophosphate,
- Bone morphogenetic protein 4 (BMP-4),
- vascular endothelial growth factor (VEGF),
- 2-mercaptoethanol,
- transforming growth factor beta 1 (TGF-β1),
- sodium pyruvate,
- non-essential amino acid,
- insulin,
- human transferrin,
- selenite,
- 3-isobutyl-1-methylxanthine (IBMX),
- indomethacin,
- α-Melanocyte Stimulating Hormone (α-MSH),
- Hydrocortisone,
- Stem Cell Factor (SCF),
- Nerve Growth Factor beta (NGF-β),
- Hepatocyte Growth Factor (HGF),
- 5-azacytidine (5-aza),
- suberoylanilide hydroxamic (SAHA), and/or
- bone morphogenetic protein-2 (BMP-2).

In another preferred embodiment, the first medium does not contain any one of the above-listed differentiation-inducing agents in an amount sufficient to induce differentiation of the cells. In another preferred embodiment the first medium does not contain any one of the above-listed differentiation-inducing agents.

The first medium comprises a nutrient base, e.g. Dulbeccos Modified Eagle Medium base (DMEM). It may further comprise serum, preferably human serum. In another preferred embodiment, the first medium further comprises basic fibroblast growth factor (bFGF) and epidermal growth factor (EGF). The first medium may further comprise insulin, human transferrin and/or selenite.

The inventors found that the medium-air interface encapsulates the hair follicle within a thin film of medium, which then holds the follicle by the means of surface tension and prevents it from floating, attaching it to the membrane, which facilitates cell outgrowth and migration. For this reason 0.7 ml to 1.1 ml, preferably 0.8 ml to 1.0 ml, most preferably about 0.9 ml of medium is typically used for the first two days to enhance the attachment. Thereafter, greater than 1.1 ml to 1.5 ml, preferably 1.2 to 1.4 ml, most preferably about 1.3 ml is typically used to enlarge the liquid area around the hair follicles for enhancing the cell migration.

In the bottom compartment of the Transwell, i.e. the compartment beneath the membrane, a layer of feeder cells is preferably employed. The feeder layer cells are preferably human fibroblasts, more preferably human inactivated dermal fibroblasts. There is no physical contact between hair follicles and feeder cells, but they are connected by the medium. The feeder cells release nutrition and growth factors into the medium, which are utilized by the hair follicle and out-growing cells, which to some extent mimicks an environment of dermal mesenchymal cells that favors the cell outgrowth.

Preferably, the isolation and cultivation of the cells is done under hypoxic conditions. 5% O₂ may for example be used during the isolation and cultivation process. In one embodiment, step (iii) is carried out under hypoxic conditions. In another embodiment, step (iv) is carried out under hypoxic conditions. In yet another embodiment, both step (iii) and step (iv) are carried out under hypoxic conditions.

After about 7 days, cells migrate out from the hair follicle ORS onto the membrane and form a monolayer. When the monolayer is expanding and closely to confluent, the upper chamber is filled with first medium or another suitable MSCORS isolation medium, and the cells are allowed to grow for about two further days. The two days' immersion culture with medium on top of Transwell is to boost the cell proliferation by providing abundant medium with whole membrane area to grow. MSCORS so rapidly migrate out of the hair follicle ORS and form a layer, with firm substrate support from the membrane (e.g. a PET membrane). Hair follicles undergoing the procedures described herein produce cell layers with a larger surface.

The culture of step (iii) is typically carried out for 14 to 25 days, preferably for 15 to 24 days, more preferably for 16 to 23 days, more preferably for 17 to 22 days, most preferably for about 18 or about 19 or about 20 or about 21 days.

Preferably upon confluence, typically within about 3 weeks cultivation, the cells are harvested from the membranes, to obtain a cell suspension. A preferred harvesting method using stepwise trypsinization is as follows: The medium is aspirated on both sides of membrane (upper and lower compartment), and pre-warmed PBS is added; the cell layer and hair shafts are gently rinsed and the washing step is repeated 3 times; 0.5ml of 0.04%/0.03% Trypsin/EDTA is added onto the membrane, and incubated for 6-8 mins with occasional microscope observation; when many of the cells contract into a sphere shape, the 6 well plate is gently tapped, and the cell layer is gently rinsed by pipetting. The supernatant is collected and aspirated into a 15ml conical tube with 0.5ml FBS to neutralize the reaction. Another 0.5 ml Trypsin/EDTA is added into the Transwell, and the trypsinization process is repeated 2-3 times, until all of the cells on the membrane are detached. All the supernatant together with cells is collected into the same 15ml conical tube with 0.5ml FBS to neutralize the reaction.

Preferably stepwise trypsinization means that the digestion process continues with 2 to 3 times until all of the cells are harvested, and collected together into the same 15 ml tube + PBS. Because, after 3 weeks of outgrowth, cell layer attachment is very tight, therefore one time of trypsinization cannot harvest all of the cells. On the other hand, a longtime exposure to trypsin is harmful for the cells. Therefore the "stepwise trypsinization" is used to reduce the cell damage while harvesting all the cells.

Therefore, the inventors designed the trypsinization as several short steps of 6-8 min, wherein after one step of trypsin digestion, the detached cells are released into the trypsin solution, and then transferred to a tube with FBS, and reaction is neutralized and stops. The undetached cells still remain on the membrane, and by adding new trypsin, the digestion process continues, until they are eventually detached.

This "stepwise" step provides longer time to those stable cells that requires more digestion, and also protect the cells from over-long trypsin damage.

The harvested cell suspension is then transferred to a solid support, e.g. a polystyrene culture dish or culture flask. The solid support is preferably coated with a polymer which facilitates attachment and proliferation. Suitable support are e.g. Advanced TC™ culture dishes and flasks. After the transfer the cells are further cultured in the presence of the second medium promoting proliferation of the MSCs without promoting their differentiation (hereinafter referred to as "second medium"), according to step (iv) of the method of the invention.

A preferred transfer protocol after the harvesting is as follows: Cells are centrifuged and resuspended in second medium and seeded onto the Advanced TC™ 6-well plate. After 24 hours of attachment, the non-attached cells are washed away, and the attached cells are proceeded to normal cell culture. Medium is changed twice a week, and cells are subcultured to standard T25 or T75 cell culture flasks.

The second medium comprises a nutrient base, e.g. Dulbeccos Modified Eagle Medium base (DMEM). It may further comprise serum, preferably human serum. In another preferred embodiment, the second medium further comprises basic fibroblast growth factor (bFGF) and epidermal growth factor (EGF). The second medium may further comprise insulin, human transferrin and/or selenite. Preferably, the second medium comprises interleukin-6 (IL-6). In a specific embodiment the first medium and the second medium are identical.

The culture of step (iv) is typically carried out for 21 to 35 days.

The method of the invention is very efficient in that it allows the generation of a high number of MSCs from a given number of hair follicles within few weeks. Typically, the number of MSCs obtained from step (iv) is at least 10⁴ per epilated hair, preferably at least 2x10⁴ per epilated hair, more preferably at least 5x10⁴ per epilated hair, most preferably at least 1x10⁵ per epilated hair or at least 1.5x10⁵ per epilated hair. In one embodiment, not more than 100 epilated hairs are processed in accordance with the method of the invention, and the number of MSCs obtained from step (iv) is at least 10⁷, preferably at least 1.5x10⁷. In another embodiment, not more than 60 epilated hairs are processed in accordance with the method of the invention, and the number of MSCs obtained from step (iv) is at least 6x10⁶, preferably at least 9x10⁶. In yet another embodiment, 40 to 60 epilated hairs are processed in accordance with the method of the invention, and the number of MSCs obtained from step (iv) is at least 4x10⁶, preferably at least 6x10⁶.

The MSCs obtainable by the method of the invention are positive for the markers CD73, CD90 and CD105, and are negative for the markers CD34, CD45, CD11b, CD19 and HLA-DR. In a preferred embodiment, the MSCs of the invention are further positive for the markers CD44, CD133, CD13, CD71, Nestin, N-Cadherin, Fibronectin, Vimentin, Integrin α5 (CD49e) and Integrin αV (CD51). The MSCs of the present invention fulfill the criteria defined by the International Society for Cellular Therapy (ISCT) (Cytotherapy (2006) Vol. 8, No. 4, 315-317). Accordingly, the MSCs of the present invention have the following properties 1 to 3:

| **Minimal Criteria for Identifying MSC ISCT** | | |
|---|---|---|
| 1*. In vitro* Cultivation | Adherence to plastic in standard culture conditions | |
| | | |
| 2. Surface Marker | Positive (95%) | Negative (≤2%) |
| | CD105 | CD45 |
| | CD73 | CD34 |
| | CD90 | CD14 or CD11b |
| | | CD79α or CD19 |
| | | HLA-DR |
| | | |
| 3. *In vitro* Mesoderm | Osteoblasts | |
| Differentiation: | Adipocytes | |
| | Chondroblasts | |

In a further aspect of the invention the MSCs obtained from step (iv) are differentiated into various target cells. Examples of differentiated cells include, but are not limited to, cardiomyocytes, chondrocytes, osteoblasts, adipocytes, endothelial cells and smooth muscle cells. For differentiation the MSCs are cultured in the presence of one or more agents promoting the differentiation into the respective target cell. Suitable agents promoting the differentiation into the respective target cells are known to the skilled person.

For example, for differentiation into osteoblasts, the MSCs may be cultured in the presence of dexamethasone, ascorbic acid, and β-glycerophosphate. The culture medium for differentiation may further comprise serum, e.g. human serum or fetal bovine serum and/or L-glutamine.

For differentiation into chondrocytes, the MSCs may be cultured in the presence of TGF-β1, BMP-4, dexamethasone, ascorbic acid, sodium pyruvate, insulin, transferrin and selenite in a high cell density of pellet culture. The culture medium for differentiation may further comprise serum and/or non-essential amino acids, and/or L-glutamine.

For differentiation into endothelial cells, the MSCs may be cultured in the presence of VEGF. The culture medium for differentiation may further comprise serum, BMP-4, 2-mercaptothanol and/or L-glutamine.

For differentiation into smooth muscle cells, the MSCs may be cultured in the presence of TGF-β1. The culture medium for differentiation may further comprise L-glutamine and serum.

For differentiation into adipocytes, the MSCs may be cultured in a commercially available adipogenic differentiation medium, e.g. StemPro Adipogenesis Differentiation Kit (Thermo Fisher Scientific). Apart from the commercially available differentiation formula, an alternative culture medium for adipogenic differentiation may comprise insulin, human transferrin, selenite, 3-isobutyl-1-methylxanthine (IBMX), and indomethacin.

For differentiation into cardiomyocytes, the MSCs may be cultured in the presence of 5-azacytidine (5-aza), suberoylanilide hydroxamic acid (SAHA), bone morphogenetic protein-2 (BMP-2) and 2-mercaptothanol. The culture medium for differentiation may further comprise serum, e.g. human serum or fetal bovine serum and/or L-glutamine.

It was unexpectedly found by the inventors that the differentiation into osteoblasts, endothelial cells and smooth muscle cells was more efficient for the MSCs of the invention than for adipose tissue-derived MSCs. For example, significantly higher mineral deposition and alkaline phosphatase activity was found for osteoblastsderived from MSCORS; significantly higher cumulative anastomosis length in endothelial cells derived from MSCORS; and prominently elongated cell soma in smooth muscle cells differentiated from MSCORS (See example 4). Therefore, in a preferred embodiment the method of the invention comprises differentiating the MSCs obtainable or obtained from step (iv) into osteoblasts, endothelial cells or smooth muscle cells.

In another aspect the invention relates to a population of MSCs obtainable by the method described herein.

In another aspect the invention relates to the use of MSCs obtainable by the method described herein for generating differentiated cells, preferably differentiated cells as defined herein above.

In yet another aspect the invention relates to a mesenchymal stem cell obtainable by the method described herein for use for purposes of aesthetic, structural or functional improvement of healthy tissues, organs or organ systems as well as for the treatment of a disorder affecting tissues, organs or organ systems that may be structurally or functionally related to the mesenchyme, particularly in the following embodiments. In one embodiment the disorder is a bone disorder, e.g. a bone injury. In another embodiment, the disorder is a disorder affecting the cartilage, e.g. osteoarthritis. In another embodiment, the disorder is a cardiovascular disorder. In another embodiment, the disorder is a wound.

In further aspects the present invention relates to the following:
The use of mesenchymal stem cells obtainable by the method described herein for generating conditioned medium containing MSC-synthetized mediators.
The use of mesenchymal stem cells obtainable by the method described herein for generating and extracting exosomal content containing MSC-synthetized mediators.
A mesenchymal stem cell obtainable by the method described herein.
A conditioned medium as described above.
An exosomal content as described above.
A conditioned medium as described above for use in the treatment of a disorder selected from the group consisting of degenerative, inflammatory, immune, autoimmune, malignant, acute or chronic disorders and injuries of cells, tissues, organs or organic systems functionally or structurally related to mesenchyme.
A conditioned medium as described above for use in the revitalization and regeneration of cells, organs, tissues, organic systems.
A conditioned medium as described above for use in replacing one or more of the differentiated tissues as defined above.
An exosomal content as described above for use in the treatment of a disorder selected from the group consisting of degenerative, inflammatory, immune, autoimmune, malignant, acute or chronic disorders and injuries of cells, tissues, organs or organic systems functionally or structurally related to mesenchyme.
An exosomal content as described above for use in the revitalization and regeneration of cells, organs, tissues, organic systems.
An exosomal content as described above for use in replacing one or more of the differentiated tissues as defined above.

### EXAMPLES

### Example 1: Isolation, purification and proliferation of hMSCORS

**Human hMSCORS Isolation Medium ('First Medium'):**

| | |
|---|---|
| DMEM (Low Glucose) | up to 88% |
| Human Serum | 10% |
| ITS Premix | 1% |
| bFGF | 10ng/ml |
| rhEGF | 20ng/ml |
| L-Glutamine | 2mM |
| Pen/Strep | Penicillin 100U/ml, Streptomycin 100µg/ml, 1% v/v from Sigma-Aldrich supplied concentration |

Human serum was employed throughout the isolation procedure.

**Human hMSCORS Cultivation Medium (Second Medium'):**

| | |
|---|---|
| Human hMSCORS | |
| DMEM (Low Glucose) | up to 89% |
| Bovine Serum | 10% |
| bFGF | 10ng/ml |
| rhEGF | 20ng/ml |
| L-Glutamine | 2mM |
| IL-6 | 10ng/ml |
| Pen/Strep | Penicillin 100U/ml, Streptomycin 100µg/ml, 1% v/v from Sigma-Aldrich supplied concentration |

### Sampling and Processing

The hair donor restrained from washing hair for at least 48 hours, to prevent the loss of sebaceous fat and hair follicle contracting, which would lead to reduced amount of the ORS tissue plucked out along with hair shafts. The hairs of the temporal region were held by forceps about 2 mm away from the root and plucked in the direction of hair growth. The hair follicle was plucked along with the hair shafts, providing the most of the native ORS tissue. This procedure was carried out for 45 hair follicles from the same donor (16,0875 ± 2,95 mg, tissue weight, counting interpersonal variation).

Plucked hair follicles were immersed and stabilized in DMEM with antibiotics and L-Glutamine for 2-4 hours at room temperature. Scalp grease, dandruff and debris were washed away in several steps using this solution. After rough rinse with PBS with antibiotics, the dermal papilla of hair follicles were excised under the microscope. Hair follicles were intensively rinsed 10 times using PBS with antibiotics, 10 min each time with vigorous shaking at 300 rpm.

Washed hair follicles were digested with 5mg/ml Collagenase X with antibiotics for 12 min at 37°C.

### MSCORS Isolation

Digested hair follicles were carefully rinsed with PBS and seeded onto the Transwell membrane inserts (Corning). The inserts were assembled into Corning Cellstar 6-well plate, with human fibroblast feeder layer cultivated on the lower side of the membrane. 0.9 ml of medium was filled from underneath Transwell inserts, and the prepared hairs were incubated at 37°C, under 5% hypoxic conditions. After two days, medium was changed for the first time with 1.3 ml volume, and the hair follicles were documented using a microscope. Medium was further replenished two times a week, preceded by gentle washing with pre-warmed PBS.

After 7 days, cells started to migrate out of the hair follicles ORS onto the Transwell membrane, and within 2-3 weeks, the cells formed a monolayer around the hair shafts on the membrane.

### MSCORS Harvest

After 3 weeks, MSCORS were harvested by trypsinization: the medium was aspirated and the cells on the membrane were gently rinsed 3 times using pre-warmed PBS.

0.5 ml 0.04%/0.03% Trypsin/EDTA was added onto the cell layer in the Transwell chamber, and incubated for 6 mins in 37°C with occasional microscopic visual observation. Most of the detached cells became round; at this point, the cell vessel was gently tapped and all the cells were aspirated into a 15ml conical tube with 0.5ml FBS to neutralize the trypsinisation reaction. This step was repeated with 2 min incubation afterwards until all the cells were detached from the Transwell membrane. Cell suspension was collected into the tube with the FBS. Cell suspension was centrifuged at 220g for 3 min, resuspended with culture medium, and the cells were seeded onto 6-wells cell culture dish.

### MSCORS Cultivation:

After 24 hours, around 30% of target cells were attached to the plastic surface of culture vessel, and over 60% cells were not attached. After PBS washing, fresh medium (Second Medium) was added. Cell divided and grew in the colony pattern in the course of the next 5 days, and at this point they started to grow out onto 2D plastic culture surface. When the cells reached 80-90% confluence, they were trypsinised and subcultured in an uncoated cell culture vessel.

A cell doubling time of less than 8 days was determined for the MSCORS.

### Example 2: Characterization of hMSCORS

MSCORS were characterized with immunofluoresence staining, which showed a correct MSC profile and differentiation potentials. Controlled with Adipose-MSC, MSCORS showed comparable or higher marker expression of CD44, CD133, CD13, CD71, Nestin, N-Cadherin.

**Table 1. Results of MSC marker FACS analysis:**

| | **CD44** | **CD133** | ***CD34** | **CD13** | **CD71** | ***CD31** |
|---|---|---|---|---|---|---|
| **MSCORS** | **++** | **+** | **-** | **++** | **++** | **-** |
| **ADMSC** | ++ | - | - | **+** | + | - |

| | | | | | | |
|---|---|---|---|---|---|---|
| * Negative Marker of MSC | | | | | | |

**Table 2. Results of MSC marker FACS analysis:**

| | **Nestin** | **N-Cadherin** | **Fibronectin** | **Vimentin** | **Integrin α5 (CD49e)** | **Integrin αV (CD51)** |
|---|---|---|---|---|---|---|
| **MSCORS** | **++** | **+** | **++** | **+** | **+** | **+** |
| **ADMSC** | + | - | ++ | + | ++ | + |

FACS-based analysis using MSC markers according to the minimal criteria for defining multipotent MSC by the International Society for Cellular Therapy (ISCT) revealed that MSCORS were expressing MSC markers in accordance with the ISCT MSC Criteria on a protein level. MSCORS are expressing high levels of positive MSC markers CD105, CD73, CD90, CD44, whereas they do not express negative markers CD19, CD11b, CD14, CD34, CD45, CD79a and HLA-DR surface markers. The successful procedure endows MSCs with a correct expression profile, comparable to that of the adipose MSCs according to ISCT criteria. Summarized results are provided in the table below and the FACS analysis plots further below.

**Table 3. Results of MSC marker FACS analysis:**

| | MSC Positive Marker | | | MSC Negative Marker | | | | |
|---|---|---|---|---|---|---|---|---|
| | CD73 | CD90 | CD105 | CD34 | CD45 | CD11b | CD19 | HLA-DR |
| MSCORS | + | + | + | - | - | - | - | - |
| ADMSC | + | + | + | - | - | - | - | - |

| | | | | | | | | |
|---|---|---|---|---|---|---|---|---|
| Plots of FACS analysis of positive and negative MSC markers in MSCORS (A) compared to ADMSC (B) are shown in Figure 1. | | | | | | | | |

### Gene expression profiles

According to the gene expression profile/mRNA copy assessment based on the qRT-PCR analysis, MSCORS are expressing CD44, and high levels of CD73, CD90, CD105. The expression levels of the analyzed markers are increasing with the duration of the MSC-directed cultivation for the entire span of the expression monitoring (in the course of five cultivation passages, based on 3 biological experiments with technical replicates, single proband).

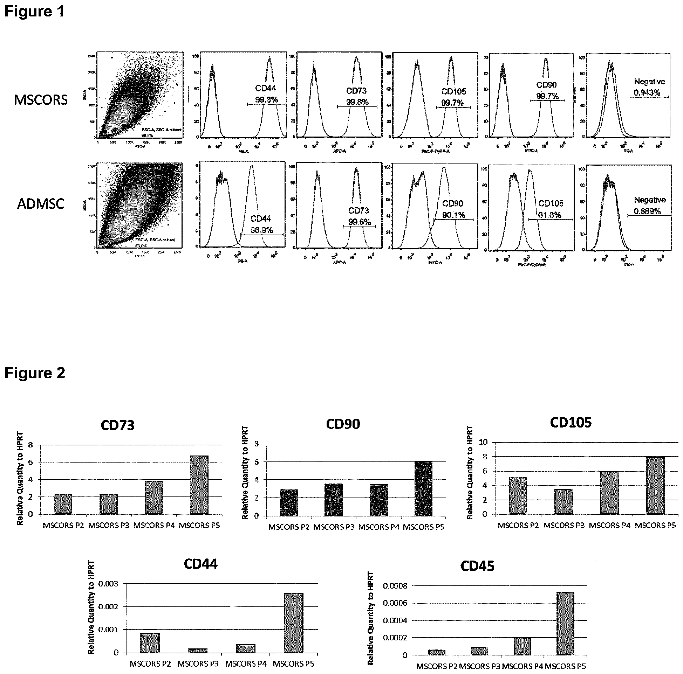

The results of the gene expression data are shown in Figure 2.

### Example 3: Comparison with prior art methods

In the following references, methods for isolating stem cells from hair follicles are described:
- Li et al. (2015) Cell Tissue Res 362: 69-82 (hereinafter referred to as D10)
- Zhang et al. (2013) International Journal of Molecular Medicine 31: 913-921 (hereinafter referred to as D11)
- Wang et al. (2013) Stem Cell Rev and Rep 9: 451-460 (hereinafter referred to as D12)

The method of the present invention differs from the methods described in D10-D12 at least in that the bulb of the epilated hair is removed, a collagen-degrading treatment is applied, and the follicles are first cultured on a liquid-permeable membrane with a first medium and thereafter on a solid support with a second medium as defined in the claims.

The methods of D10-D12 were compared with the method of the invention, as described in the following.

For each experiment, a total of 45 hairs were plucked from 3 human test persons, 15 per person. The follicles were processed exactly as described in D10, D11, D12 or in Example 1 above, respectively. It turned out that only very few follicles which were processed according to D10-D12 resulted in outgrowth of cells. Upon 4 weeks of culture the following results were obtained:

**Table 4.**

| | D10 | D11 | D12 | Present invention |
|---|---|---|---|---|
| Number of follicles with outgrowth of cells by 4 weeks | 1 | 0 | 1 | 32 |
| Number of cells obtained by P0 | 50,000 | 0 | 210,800 | 4,317,100 |
| Confluence reached after 4 weeks of culture? | No | No | No | Yes |

The interpretation of this surprising result is as follows. For isolating/expanding the MSCs, D10, D11 and D12 teach placing the follicles directly onto a cell culture (plastic) support. In the above-described tests this almost never worked, as outgrowth directly from the follicle onto the cell culture plastic was observed only in very few cases. It thus appears that the reports D10, D11 and D12 have been based solely on isolated single successful cases (rare follicles that yielded outgrowth), whereas the non-outgrowing follicles were not reported in D10, D11 and D12.

The immersion culture with medium on top of Transwell supports cell proliferation by providing abundant medium with whole membrane area to grow. With this procedure, MSCORS rapidly migrate out of the hair follicle ORS and form a layer, with firm substrate support from the membrane, medium-air interface stimulation, nutrition supply from medium and feeder layer. It is evident that hair follicles processed according to the present invention produce cell layers with a larger surface.

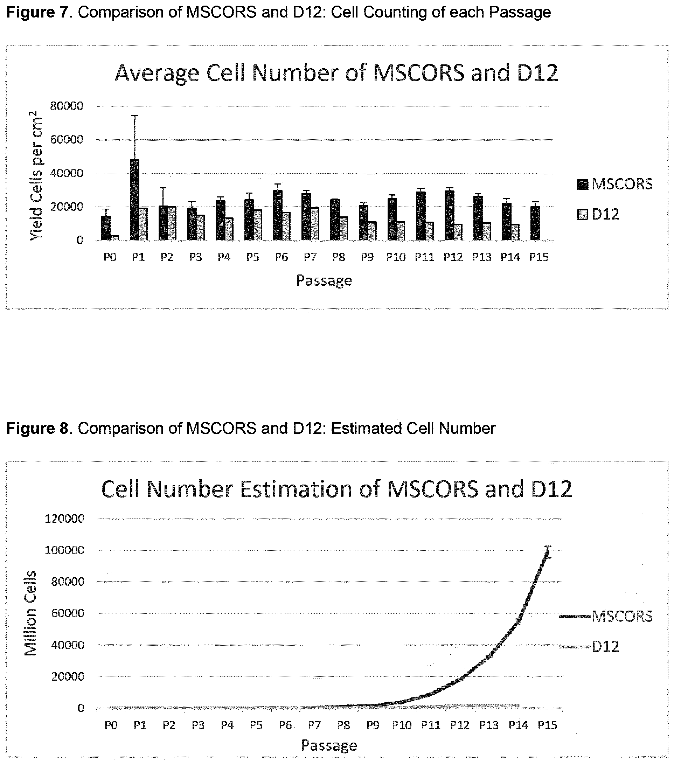

After passage 0, the present invention produced a cell culture of MSCORS with stable proliferation and subculturing passages from all three donors, whereas only one sample from method D12 produced a cell culture, with distinctly lower cell number, which is presented in Figure 7 and Figure 8. There was only one hair follicle in D10 and D12 with cell outgrowth. However after subculture to passage 0, these cells failed to proliferate, remained senescent and apoptosed rapidly. The cell numbers during continuous passage in accordance with this invention and according to D12 are shown in Figure 7. The projected cell number estimation based on cell counting in each passage is shown in Figure 8.

In addition, the proximal part of the hair shaft was not removed in D10, D11 and D12. From the Figures in D10, D11 and D12, it was very clear that the cells migrated from the hair bulb onto the culture surface. Fibroblasts from dermis, also known as Dermal Papilla cells (DP cells) from dermal papilla, play an important role in hair shaft growth and hair matrix production. The rests of dermis and dermal papilla are frequently carried over along with the proximal part of the hair follicle. These fibroblasts are known to express some MSC markers, but they are limited in their cell differentiation capacities. Also, judging by the incomplete expression profile and low differentiation potentials, the "hf-MSC" in D10 are in all probability dermal fibroblasts. Fibroblasts share very similar properties with MSC in expression profile and differentiation potentials, and it can be difficult to differentiate the two populations. This has been documented in the current literature: Hematti, P. (2012) Cytotherapy 14(5): 516-521. Denu, R. A., et al. (2016) Acta Haematol 136(2): 85-97. Alt, E., et al. (2011) Biol Cell 103(4): 197-208.

It is also noted that D10-D12 do not show a full MSC immunophenotyping according to the ISCT criteria (Cytotherapy (2006) Vol. 8, No. 4, 315-317).

In summary, the methods of D10-D12 do not allow generating a sufficient number of MSCs from a given number of hair follicles. In addition, there is reasonable doubt as to whether the cells isolated and expanded in D10-D12 were actually MSCs. It appears likely that a significant portion of the cells were fibroblasts.

### Example 4: Differentiation of MSCORS

Cultivated MSCORS were subjected to differentiation procedures towards osteoblasts, chondrocytes, adipocytes, smooth muscle cells and endothelial cells. Adipose tissue-derived MSCs (ADMSC) were used as control because they are considered a standard for low-invasively attained MSCs, having in mind the relatively low- invasive nature of liposuction in comparison to the painful biopsy of bone marrow or synovial fluid or discomforting blood extraction. Nevertheless, it needs to be acknowledged that liposuction requires hypodermal invasion with a thick suction needle as well as constant agitation of the fat tissue in the course of liposuction; its invasivity grade is therefore higher and not comparable to the non-invasive hair plucking used to acquire hair follicles.

### Methods

### Osteogenic Differentiation

6x10⁴ cells were seeded into a 24-well plate, and cultivated for 3 weeks or for 4 weeks using MSC Osteogenic Medium. Cells were examined with ALP activity assay, Alizarin Red staining, Calcium-phosphate assay.

**MSC Osteogenic Medium:**

| | |
|---|---|
| DMEM | to 90% |
| FBS | 10% |
| Dexamethasone | 2 x 10⁻⁷ M |
| Ascorbic Acid | 50ug/ml |
| β-giycerophosphate | 10mM |

### Chondrogenic Differentiation:

2.5x10⁵ cells were centrifuged into pellet in 15ml conical tube in 800g for 5 min, and cultivated with MSC Chondrogenic Medium for 3, 4 and 5 weeks. Differentiated cartilage tissue was examined with histological staining, including H&E, Alcian Blue, Safranin O, Collagen I and Collagen II.

**MSCORS Chondrogenic Medium:**

| | |
|---|---|
| DMEM/F12 | up to 97% |
| Human Serum | 1% |
| ITS Premix | 1% |
| TGF-β1 | 10ng/ml |
| BMP-4 | 10ng/ml |
| Dexamethasone | 10⁻⁷ M |
| Ascorbic Acid | 50ug/ml |
| Na Pyruvate | 50ug/ml |
| Non-essential AA | 1% |

### Adipogenic Differentiation

2x10⁴ cells were seeded into 24-well plate, and cultivated for 3 weeks or 4 weeks using MSC commercially available adipogenic differentiation medium, StemPro™ Adipogenesis Differentiation Kit (Thermo Fisher Scientific). Intracellular lipid vesicles in mature adipocytes were detected by Oil Red staining.

### Optional (home-made adipogenic differentiation medium):

**Adipogenic Differentiation Medium:**

| | |
|---|---|
| DMEM (1g/L Low Glucose) | up to 88% |
| Fetal Bovine Serum | 10% |
| L-Glutamine | 1% v/v 2mM |
| Non-essential AA | 1% |
| Dexamethasone | 1µM |
| 3-isobutyl-1-methylxanthine (IBMX) | 500µM |
| Indomethacin | 100µM |
| Insulin | 10µm/ml |

### Endothelial Differentiation

2.4x10⁵ cells were seeded on 1 well of a 6-well plate, and cultivated using MSCORS Endothelial Medium for 3 and 4 weeks. Cells were stained with a CD31 antibody, tested using Matrigel based Angiogenic Assay and analyzed by the means of ImageJ Software.

**MSCORS Endothelial Medium:**

| | |
|---|---|
| DMEM (Low Glucose) | to 94% |
| FBS | 5% |
| L-Glutamine | 1% v/v 2mM |
| 2-Mercaptoethanol | 0.5mM |
| VEGF | 30ng/ml |
| BMP-4 | 5ng/ml |

### Smooth Muscle Differentiation

MSC before passage 4, were cultivated in DMEM+10% FBS medium until reaching 80% confluence. Cells were detached using Trypsin/EDTA, and subcultured onto a new cell culture vessel at density of 1.5X10⁵/cm². Hereby, 3x10⁵ cells were seeded into a well of a 6-well plate. After two days of cultivation, culture medium was changed to Smooth Muscle Differentiation Medium, and differentiation was continued for 3 weeks and for 4 weeks. After the targeted time point, differentiation-induced cells were harvested and subjected to immunofluorescence staining with alpha smooth muscle actin (αSMA) antibody.

**MSCORS Smooth Muscle Medium:**

| | |
|---|---|
| DMEM (1g/L Low Glucose) | up to 89% |
| Fetal Bovine Serum | 10% |
| L-Glutamine | 1% v/v 2mM |
| TGFβ-1 | 10ng/mL |

**Cardiomyocyte Differentiation**

| | |
|---|---|
| 5-azacytidine (5-aza) | 5 µM, for 24h, then to 1 µM 5-aza for 7 days |
| suberoylanilide hydroxamic (SAHA) | 10 µM for 24h, then to 1 µM SAHA for 7 days acid |
| bone morphogenetic protein-2 (BMP-2) | 10ng/ml |
| 2-mercaptothanol | 1 mM |

### Results

### Cell number

The cell number of isolated and enriched human MSCORS from plucked hair follicles was comparable to that of ADMSC, however with minimal starting amount of material in case of MSCORS. One MSCORS isolation requires 15-60 hairs, which, due to individual differences, sums up to 5 mg - 22 mg tissue weight. ADMSC isolation requires 25g of adipose tissue to reach the same efficiency. MSCORS technology yields 1 to 5 million MSCs in 4 weeks upon isolation, which already corresponds to therapeutically relevant minimal cell amounts. Within 40 days after cell harvesting, more than 30 million cells in P5 are obtained with the cell doubling time of 7.5 days, which is within the sufficient scale for most MSC-based cell therapies.

### MSCORS are expressing human mesenchymal stem cell markers on a comparable level with adipose MSCs, or with higher intensity

MSCORS were characterized with immunofluoresence staining, and showing MSC profile and differentiation potentials. Controlled with Adipose-MSC, MSCORS showed comparable or higher marker expression of CD44, CD133, CD13, CD71, Nestin, N-Cadherin. The results are described above in Example 2.

### Chondrogenic Differentiation

MSCORS formed structurally correct pellet during the differentiation process, and the cells synthesized proteoglycans and collagen type II detected by histological staining and immunofluorescence. Extracellular matrix was intensively deposited in higher magnification. The amount and staining intensity of cartilaginous extracelluar matrix from MSCORS was comparably higher than in the ADMSC, which showed more porous structure.

### Adipogenic Differentiation

MSCORS were differentiated into mature adipocytes, with cumulative intracellular lipid vesicles. The lipids were detected by Oil Red staining. In this case, ADMSC showed superior adipogenic differentiation in comparison to the MSCORS.

### Smooth Muscle Differentiation

Differentiation into smooth muscle resulted in spindle-shaped myocytes, prevalently symplasmic, in varying sizes. Compared with ADMSC, MSCORS differentiated into more elongated myocytes with more distinctive morphology and stronger expression of alpha smooth muscle actin (αSMA) than the myocytes differentiated from the ADMSC. MSCORS-derived myocytes displayed clear myogenic fusion with multinuclear fibrous structure whereas the ADMSC-derived myocytes symplasm was less pronounced. Upon the same exposure time, the fluorescence images of the MSCORS-derived vascular smooth muscle cells (VSM) displayed a visibly stronger signal of αSMA and a more pronounced elongation of the cell soma in comparison to the ADMSC-differentiated VSM cells. After smooth muscle differentiation, MSCORS-derived show evidence of myogenic fusion with multinuclear fibrous structure within the syncytium. The MSCORS-derived SM cells appear more elongated than the ADMSC-derived cells.

To study the extent of Smooth Muscle Differentiation in MSCORS and ADMSC, we established a ImageJ-based semi-quantitative method to evaluate the αSMA actin stress fibers within the cells. After 3 and 4 weeks differentiation, cells were extremely elongated with abundant cytoskeleton with αSMA, nevertheless, it is difficult to distinguish and evaluate each individual cell from the photos, since the cells are normally overlapped and inter-connected. Therefore, fluorescent αSMA actin stress fibers were visually extracted by the means of ImageJ software, and the number and length of αSMA actin stress fibers was quantified in each representative photos of MSCORS and ADMSC.

Fluorescence images of the αSMA signal (Alexa594, Red Fluorescence) without DAPI, were analyzed using ImageJ plug-ins, "Tubeness" and "Angiogenesis Analyzer". In each image, number and length of αSMA fibers were measured. 11 images of ADMSC, and 74 images of MSCORS were analyzed (Carpentier G, Martinelli M, Courty J and Cascone I. Angiogenesis Analyzer for ImageJ. 4th ImageJ User and Developer Conference proceedings. Mondorf-les-Bains, Luxembourg. ISBN: 2-919941-18-6 : 198-201, 2012).

The semi-quantitative results of the SMA actin stress fiber analysis showed that MSCORS-derived smooth muscle cell populations contained a higher number of fiber and a higher total fiber length than the ADMSC-derived SM (after 3 weeks of differentiation: 134% higher fiber number, 258% higher fiber length, 4 weeks in:19% higher fiber number, 60% higher fiber length). This confirmed the descriptive morphological analysis that the MSCORS-derived SM cells are more elongated than the ADMSC-derived SM cells.

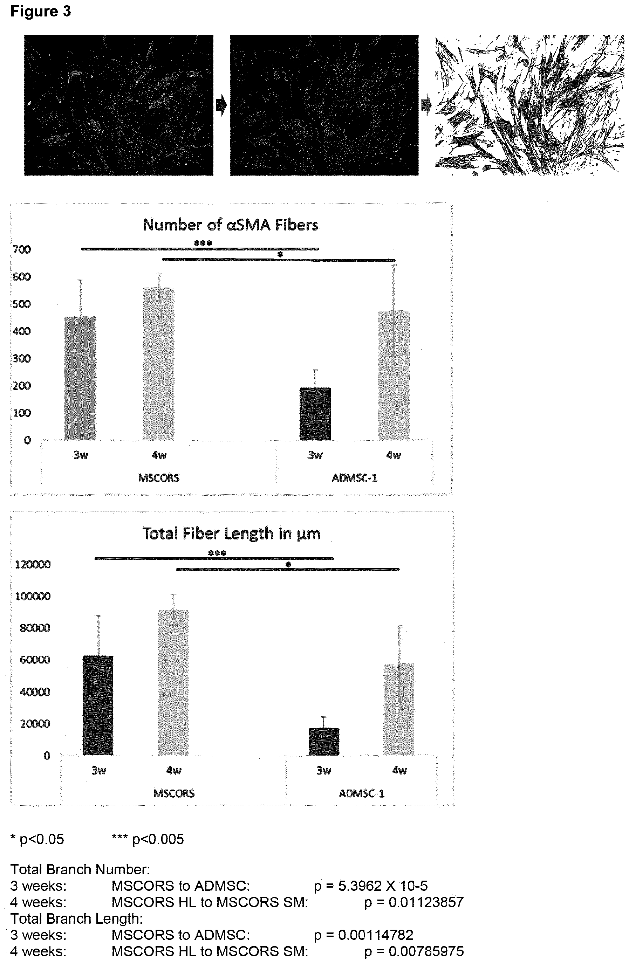

The results of the semi-quantitative are shown in Figure 3.

### Osteogenic Differentiation

MSCORS are capable of differentiating into osteoblasts, with very high level of the ALP activity and calcium deposition. Moreover, MSCORS have shown an unexpectedly efficient osteogenic differentiation, superior in comparison to that of the ADMSC. MSCORS were faster and more efficiently osteo-differentiated, showing higher ALP activity and higher deposited calcium content.

### Semi-quantitative analysis

Bright field images of ALP (dark blue) and Alizarin Red (Red) staining for osteogenesis from both MSCORS and ADMSC were semi-quantitatively analyzed using ImageJ software. The chromatic intensity of each signal were measured and quantified as Integrated Optical Density (IOD). 36 images of ADMSC osteogenesis and 200 images of MSCORS osteogenesis were analyzed.

The positive signal was analyzed by measuring the area and mean intensity of the targeted signal. Threshold of each image was determined independently by the non-positive background, and excluded during the measurement.

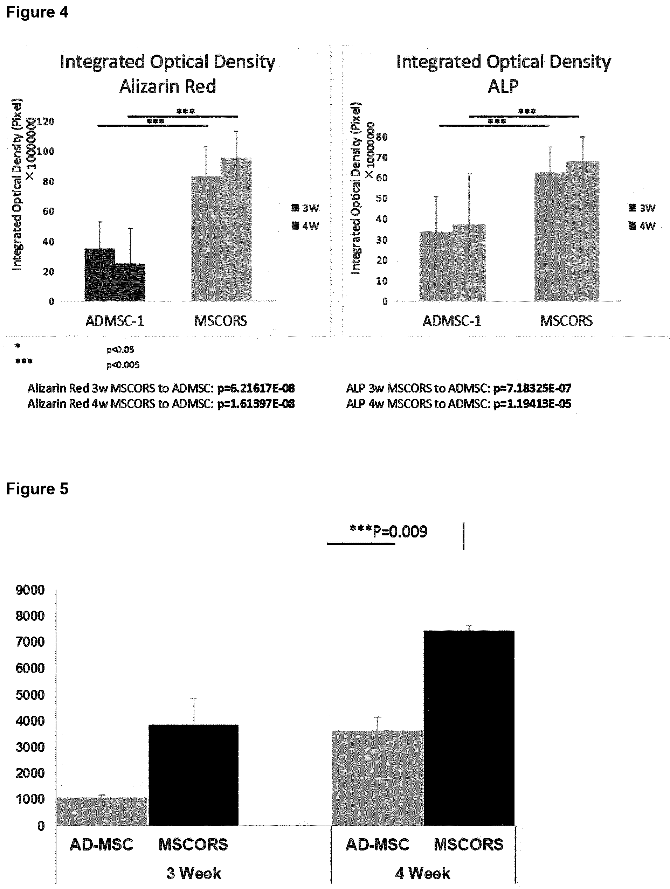

The results of the semi-quantitative analysis are shown in Figure 4.

### Quantitative analysis

Quantitative measurement of Calcium concentration in depositions was determined by the means of calcium-reacted chromogenic agent, o-cresolphthalein complexone (CPC-kit), and measured by the means of spectrophotometry. Clear trend of higher calcium content in osteodifferentiated MSCORS than the osteodifferentiated adipose MSCs was displayed. At the time point of 4 weeks, the differences in calcium content were highly significant:
The results of the quantitative analysis are shown in Figure 5.

### Endothelial Differentiation

After endothelial differentiation, cells were expressing high level of CD31, an endothelial cell marker. Results demonstrate stronger expression of CD31 protein in MSCORS-derived endothelial cells than in ADMSC-derived endothelial cells.

### Angiogenesis Assay Semi-Quantitative Analysis

Capability to form anastomotic structures is inherent to the endothelial cells, which is the base for the quantitative angiogenic test (Tube-Test). MSCORS-derived endothelial cells displayed higher potential to form anastomosis than that of the ADMSC-derived endothelial cells.

### Method of Angiogenic Assay Semi-Quantitative Analysis:

Fluorescence images of Angiogenic Assay with Calcein AM signal (Green, living cells) were analyzed using ImageJ plug-in, Angiogenesis Analyzer, which was designed by Gilles Carpentier in 2012. Several indicators of tube forming elements were analyzed, including numbers of junctions, segments, branches, and total branching length. 8 Angiogenesis images from ADMSC, and 54 images from MSCORS were analyzed.

Analysed images and semi-quantitative results demonstrated that MSCORS-derived endothelial cells displayed superior anastomotic properties compared to those of the ADMSC-derived cells, with higher number of junctions, branches and segments. Stronger Calcein AM signal from the MSCORS-derived endothelial cells indicates higher post-differentiation cell viability compared to that of the ADMSC-derived endothelial cells.

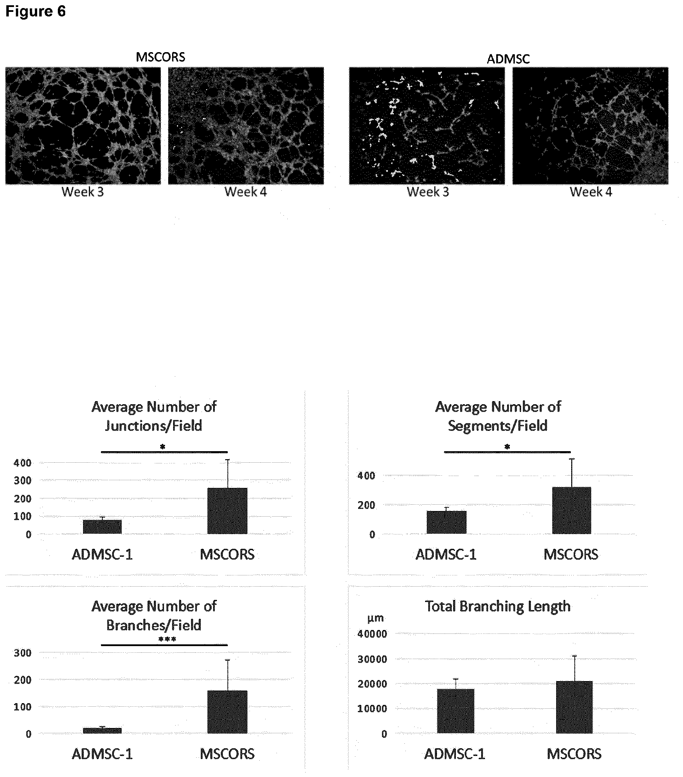

The results of the Angiogenesis Assay Semi-Quantitative Analysis in MSCORS and ADMSC are shown in Figure 6.

## Claims

1. A method for generating mesenchymal stem cells comprising the steps of:
(i) removing the bulb of an epilated hair;
(ii) incubating the remaining part of the epilated hair with a collagen degrading agent to obtain a hair follicle comprising stem cells and an at least partially degraded extracellular matrix;
(iii) culturing the hair follicle obtained from step (ii) on a liquid-permeable membrane in a first medium to allow outgrowth of the stem cells from the hair follicle, under conditions that induce proliferation of the stem cells without inducing their differentiation; and
(iv) further culturing the stem cells obtained from step (iii) on a solid support in a second medium that induces proliferation of stem cells without inducing their differentiation.

2. The method of claim 1, wherein the epilated hair is an epilated human hair, preferably a human anagen hair.

3. The method of claim 1 or 2, wherein a plurality of epilated hairs are used to generate the mesenchymal stem cells.

4. The method of claim 3, wherein at least 30 epilated hairs are used to generate the mesenchymal stem cells.

5. The method of claim 3, wherein at least 50% of the epilated hairs result in a proliferating culture of mesenchymal stem cells.

6. The method of any one of the preceding claims, wherein step (iv) comprises culturing the separated stem cells until the number of proliferated stem cells is at least 6x10⁶.

7. The method of any one of the preceding claims, wherein step (iii) is carried out for 15 to 25 days.

8. The method of any one of the preceding claims, wherein step (iv) is carried out for 21 to 35 days.

9. The method of any one of the preceding claims, wherein said first medium and/or said second medium comprises serum, preferably human serum, most preferably serum replacement.

10. The method of any one of the preceding claims, further comprising the step of (v) differentiating the proliferated stem cells obtained from step (iv) to obtain differentiated cells.

11. The method of claim 10, wherein said differentiated cells are selected from the group consisting of chondrocytes, osteoblasts, adipocytes, endothelial cells and smooth muscle cells.

12. A population of mesenchymal stem cells obtainable by the method of any one of claims 1 to 9.

13. The use of mesenchymal stem cells obtainable by the method of any one of claims 1 to 9 for generating differentiated cells.

14. The use of mesenchymal stem cells obtainable by the method of any one of claims 1 to 9 for generating conditioned medium containing MSC-synthetized mediators, and/or for generating and extracting exosomal content containing MSC-synthetized mediators.

15. A mesenchymal stem cell obtainable by the method of any one of claims 1 to 9, or a conditioned medium as defined in claim 14, or an exosomal content as defined in claim 14, for use in the treatment of a disorder selected from the group consisting of degenerative, inflammatory, immune, autoimmune, malignant, acute or chronic disorders and injuries of cells, tissues, organs or organic systems functionally or structurally related to mesenchyme; for use in the revitalization and regeneration of cells, organs, tissues, organic systems; and/or for use in replacing one or more of the differentiated tissues as defined in claim 11.
